# EUROPEAN PATENT APPLICATION

(11) **EP 4 686 446 A1**
(43) Date of publication of application: **04.02.2026**
(21) Application number: 25192685.3
(22) Date of filing: 30.07.2025
(51) Int. Cl.: A61B 5/00, A61B 5/145

(54) **MULTIMODAL ELECTRODE ARRAY FOR ELECTROCHEMICAL SENSING AND ELECTRICAL STIMULATION BY MEDICAL IMPLANTS**

(30) Priority: 31.07.2024 US 202418790074
(71) Applicant: Globus Medical, Inc., Audubon, PA 19403 (US)
(72) Inventor: TROXELL, Paden, Conshohocken, 19428 (US); PAUL, David C., Phoenixville, 19460 (US); JOHNSON, Norbert, North Andover, 01845 (US)
(74) Representative: Morabito, Sara

(57) **Abstract**

An implant device implantable within a body of a patient. The implant device includes a set of electrodes in an array spaced apart on the implant device, measurement circuitry configured to perform measurement modes on signals from the electrodes, a multiplexer that interconnects different pairs of electrodes in the array to the measurement circuitry, and a controller. The controller controls the multiplexer to selectively connect different pairs of electrodes in the array over time to perform the measurement modes that include at least two of: open circuit potential (OCP) measurement, amperometry measurement, and electrochemical impedance measurement.

## Description

### FIELD

The present disclosure relates to medical implants containing electronic circuits.

### BACKGROUND

Smart implants are traditional orthopedic implants augmented with electronics to enable sensing, therapeutic, and connectivity functions.

Typically, an electrochemical sensing system requires a dedicated electrochemical cell (i.e. set of 2 or 3 electrodes) for each sensor measurement and analyte (e.g. pH or dissolved oxygen). In some scenarios, the counter and reference electrodes can be shared, but a unique working electrode (i.e. sensing electrode) is functionalized specifically for each analyte. These existing configurations are not suitable for smart implant applications that require measurement of multiple biomarkers in a very space-constrained form factor (e.g. interbody spacer).

### SUMMARY

Embodiments of the present disclosure are directed to smart musculoskeletal implants that can provide physicians/clinicians and their patients the ability to monitor, detect, and treat different features.

Some embodiments of the present disclosure are directed to an implant device implantable within a body of a patient. The implant device includes a set of electrodes in an array spaced apart on the implant device, measurement circuitry configured to perform measurement modes on signals from the electrodes, a multiplexer that interconnects different pairs of electrodes in the array to the measurement circuitry, and a controller. The controller controls the multiplexer to selectively connect different pairs of electrodes in the array over time to perform the measurement modes that include at least two of: open circuit potential (OCP) measurement, amperometry measurement, and electrochemical impedance (EIS) measurement.

OCP measurement includes the measurement circuitry measuring free potential through a first pair of electrodes while the controller does not apply current between the pair of electrodes. Amperometry measurement includes the controller applying power to one of the electrodes in a second pair of electrodes while the measurement circuitry measures current resulting at the other electrode in the second pair of electrodes. EIS measurement includes the controller applying an alternating current to one of the electrodes in a third pair of electrodes while the measurement circuitry measures current resulting at the other electrode in the third pair of electrodes.

Some other embodiments of the present disclosure are direct to a method performed by a controller of an implant device implantable within a body of a patient. The method includes controlling a multiplexer to selectively connect different pairs of electrodes, of a set of electrodes spaced apart on the implant device, to measurement circuitry over time to perform measurement modes. The measurement modes include at least two of: open circuit potential (OPC) measurement where measurement circuitry of the implant device measures free potential through a first pair of electrodes while the controller does not apply current between the pair of electrodes; amperometry measurement where the controller applies power to one of the electrodes in a second pair of electrodes while the measurement circuitry measures current resulting at the other electrode in the second pair of electrodes; and electrochemical impedance spectroscopy (EIS) measurement, where the controller applies an alternating current to one of the electrodes in a third pair of electrodes while the measurement circuitry measures current resulting at the other electrode in the third pair of electrodes.

### DESCRIPTION OF THE DRAWINGS

Aspects of the present disclosure are illustrated by way of example and are not limited by the accompanying drawings. In the drawings:
Figure 1 illustrates an example of a smart implant system configured to alert a clinician of biofilm growth and treat the biofilm to help prevent infection, according to some embodiments of the present disclosure;
Figure 2 illustrates an example of the smart implant system for total knee arthroplasty (TKA), according to some embodiments of the present disclosure;
Figure 3 illustrates a plurality of electrodes in an electrode array, according to some embodiments of the present disclosure;
Figures 4A-4D illustrates different locations where sets of electrodes in an array may be located on the smart implant system for TKA, according to some embodiments of the present disclosure;
Figure 5 illustrates an embodiment of a smart musculoskeletal implant that includes a control and communication module that is assembled to, integrated with, or part of a traditional musculoskeletal implant, according to some embodiments of the present disclosure;
Figure 6 illustrates an example of a control and communication module, according to some embodiments of the present disclosure;
Figure 7 illustrates a three-electrode and two-electrode configuration, according to some embodiments of the present disclosure;
Figures 8-9 illustrate examples of a smart implant with three-electrode configurations with functionalized working electrodes, according to some embodiments of the present disclosure;
Figures 10-11 illustrate examples of a smart implant with a multimodal working electrodes and separate electrode arrays for sensing and stimulation, according to some embodiments of the present disclosure;
Figures 12-13 illustrate examples of a smart implant with a multimodal working electrode and separate electrode arrays for measuring/sensing and stimulation, according to some embodiments of the present disclosure;
Figures 14-15 illustrate a smart implant with a plurality of multimodal working electrodes and the same electrode arrays for measuring/sensing and stimulation, according to some embodiments of the present disclosure;
Figure 16 illustrates an electrode array that is controlled to perform measurement modes sequentially, according to some embodiments of the present disclosure;
Figure 17 illustrates an example of an electrode array that performs open circuit potential (OCP), amperometry, and electrochemical impedance spectroscopy (EIS) in series, according to some embodiments of the present disclosure;
Figure 18 illustrates an example of an electrode array that performs two electrochemical techniques in parallel, according to some embodiments of the present disclosure;
Figure 19 illustrates an example of an electrode array that performs EIS and OCP in parallel, according to some embodiments of the present disclosure;
Figure 20 illustrates an example of the controller controlling a multiplexer to select a new working electrode to perform a series of electrochemical techniques, according to some embodiments of the present disclosure;
Figure 21 illustrates a flowchart of a measurement process where electrodes in an electrode array are cycled through, according to some embodiments of the present disclosure;
Figure 22 illustrates electrodes in an electrode array be cycled through during the measurement process of Figure 21, according to some embodiments of the present disclosure;
Figure 23 illustrates a flowchart of a stimulation sequence used in conjunction with a measurement sequence, according to some embodiments of the present disclosure;
Figures 24A-24I illustrate a smart interbody spacer, according to some embodiments of the present disclosure;
Figure 25 illustrates a flowchart of a stimulation sequence used in conjunction with a bone growth measurement sequence, according to some embodiments of the present disclosure;
Figure 26 illustrates flowchart of a combined system of osteogenic and microbial applications, according to some embodiments of the present disclosure; and
Figure 27 illustrates an example of an implant device, according to some embodiments of the present disclosure.

### DETAILED DESCRIPTION

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

Embodiments of the present disclosure are directed to an electrochemical sensor and smart implant sensing system that enables measurement of multiple analytes with multiple electrochemical techniques using a multimodal electrode array where each electrode can serve a multitude of sensing functions.

Embodiments herein include a multimodal electrode array and smart implant system that may provide the following advantages: multiple electrochemical techniques from a single, non-functionalized working electrode in an array to avoid need for multiple dedicated working electrodes; any electrode in the array can be configured as the working electrode, reference electrode, or counter electrode to enable spatial sensing in a compact form factor; any electrode in the array can be configured as the anode or cathode to enable spatial stimulation in a compact form factor; clinical applications for electrochemical sensing of biofilm (e.g., infection) and tissue (e.g., bone growth); and clinical applications for electrical stimulation of biofilm (e.g., infection treatment) and tissue (e.g., bone growth acceleration).

Electrochemical sensing techniques are appealing for smart implant applications due to their ability to measure electrical, chemical, and biological analytes that are abundant *in vivo.*

Electrical stimulation is an appealing therapeutic mechanism for smart implant applications. Smart implants may employ electrical stimulation for treatment of infection, bone healing, pain, and cancer. The use of electrical stimulation alongside electrochemical sensing may require separate sets of electrodes for the stimulation and sensing functions; however, embodiments herein may use the same set of electrodes for the stimulation and sensing functions because it may be advantageous to minimize the number of electrodes that must be integrated within a smart implant due to the spatial limitations. Some embodiments of the present disclosure describes a smart implant system that enables measurement of multiple analytes with multiple electrochemical techniques as well as delivery of electrical stimulation with a multimodal electrode array where each electrode can serve a multitude of sensing and stimulation functions.

The present disclosure is organized in three parts: microbial applications, osteogenic applications, and combined applications. However, it should be noted that embodiments from each application may be applied in other applications.

Microbial is a term to describe microorganisms, especially bacterium that cause disease. In the context of the present disclosure, microbial applications include the detection and treatment of bacterial cells, bacterial biofilms, and bacterial infections. The primary clinical presentations of infection that are relevant to musculoskeletal surgery include periprosthetic joint infection (PJI) in joint arthroplasty and surgical site infection (SSI) in trauma and spine surgery. Both types of infection are caused by implant biofilms.

Periprosthetic joint infection (PJI) is a devastating complication of total joint arthroplasty caused by the development of bacterial biofilms on the surfaces of biologically inert implant components. PJI develops in approximately 30,000 patients in the United States each year and is the leading cause of revision total knee arthroplasty (TKA). Often, PJI is undetected until the patient reports acute symptoms of fever, inflammation, or sinus tracts which prompt closer follow-up and collection of synovial cultures. Alternatively, chronic infections may develop more slowly and only be detected following symptoms of pain or instability caused by septic loosening of the implant components. In either case, mature biofilms are typically well established before PJI is diagnosed. At this stage, antibiotic treatment is typically ineffective at eradicating the bacteria, which is protected by an extracellular polymeric substance (EPS) matrix. The standard of care for PJI consists of surgical debridement and/or component revision concurrent with 6-12 weeks of intravenous antibiotics. Some embodiments of the present disclosure includes means for detecting and treating infection-causing biofilms to eliminate the need for invasive and costly surgical interventions.

Surgical site infection (SSI) is a type of infection that occurs after surgery in any part of the body where surgery was performed. Similar to PJI, implant-related SSI is often caused by the formation of bacterial biofilms on implant components. In trauma and spine surgery, the incidence of SSI is generally greater in longer and more invasive procedures, such as femoral nailing and deformity correction. In lumbar spine fusion, the incidence of infection is 2-4%, however, studies have shown that infection is an under detected and underreported cause of failed fusion (i.e. pseudarthrosis). In fact, one study found positive intraoperative bacterial cultures in 10% of lumbar pseudarthrosis revision surgeries where infection was not suspected.

One way in which smart implants might treat infection is by eliminating the source, the bacterial biofilm, utilizing electrical stimulation. For example, through the use of a low-dose current-controlled treatment. This is described in more detail below.

Electrochemical Sensing is described in more detail below.

The basic working principle of an electrochemical sensor involves the reaction of a target substance with the surface of an electrode, which produces an electrical signal proportional to the concentration of the substance. An electrochemical sensor may include three electrodes, including a working electrode, counter electrode, and reference electrode that are connected to a data acquisition system called a potentiostat. Some techniques only require a two-electrode configuration where the reference electrode may be coupled to the counter electrode or excluded entirely. A variety of electrochemical techniques exist, which differ in methods of excitation and interpretation of the electrochemical reaction. Three of the most common electrochemical sensing techniques include open circuit potential, amperometry, and electrochemical impedance spectroscopy.

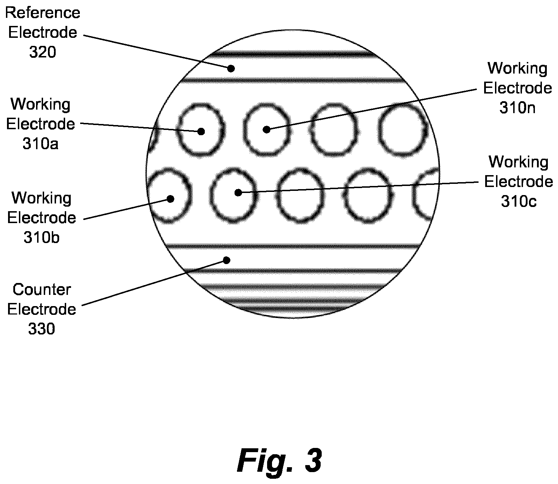

Figure 3 illustrates a plurality of electrodes in an electrode array, according to some embodiments of the present disclosure.

A working electrode (e.g., working electrodes 310a-n) includes an electrode that operate as the anode in the electrode array, and is where the sensor measurements take place. For example, the working electrodes could be utilized for spatial mapping of a single parameter, such as impedance measurement, yielding an impedance measurement at each working electrode on the electrode array. Using multiple electrochemical techniques in series, such as potentiometry, electrochemical impedance spectrometry, and cyclic voltammetry, the array of working electrodes may also be used to capture multiple characteristic readings sequentially, such as impedance, conductivity, and pH. However, in some embodiments, different electrochemical techniques could be performed on individual working electrodes in parallel. For example, cyclic voltammetry could be performed on a first working electrode, amperometry could be performed on a second working electrode (that is a different electrode than the first electrode), and impedance spectroscopy could be performed on a third electrode (that is a different electrode than the first electrode and second electrode).

A counter electrode (e.g., counter electrode 330) includes an electrode that operates as the cathode in the electrode array system.

A reference electrode (e.g., reference electrode 320) includes an electrode that is supplied a fixed reference voltage (e.g., 1.7 volts) for the sensing measurements. A reference electrode may provide a baseline against which the changes in potential at the working electrode(s) can be interpreted. By maintaining a stable and reproducible reference potential through the reference electrode, the electrode array may get more consistent electric signal response measurements so it is easier to analyze and interpret the electrical and/or chemical characteristics measured by the working electrodes.

In some embodiments, the reference electrode is not used (or is not present) on the electrode array. In yet other embodiments, the reference electrode is connected to the counter electrode thereby making them both operate as counter electrodes. How the reference electrode is used may depend on the type of electrical and/or chemical characteristic that is being measured by the working electrode(s) or the measuring process used (e.g., amperometry).

However, as described herein, any of the electrodes in an array may be selected by a multiplexer as a working electrode, counter electrode, or reference electrode during a measurement mode.

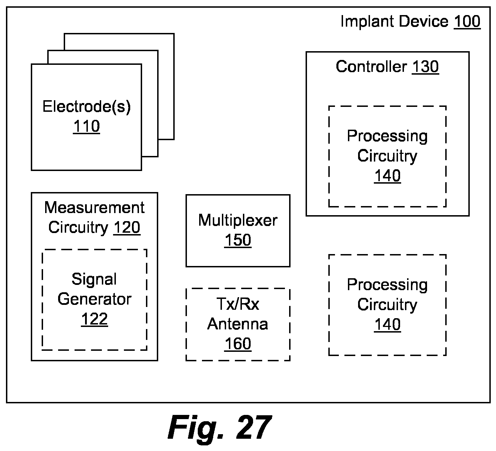

Figure 27 illustrates an example of an implant device 100 (also referred herein as an implant or smart implant), according to some embodiments of the present disclosure. The implant device 100 may be implantable within a body of a patient and include a set of electrodes 110 in an array spaced apart on the implant device 100. The implant device 100 may further include measurement circuitry 120 configured to perform measurement modes on signals from the electrodes 110. The implant device may further include a multiplexer 150 that interconnects different pairs of electrodes 110 in the array to the measurement circuitry 120. The implant device 100 may also include a controller 130 that may optionally include processing circuitry 140. Alternatively, the processing circuitry 140 is physically separate but communicatively coupled to the controller 130. The control 130 may control the multiplexer 150 to selectively connect different pairs of electrodes 110 in the array over time to perform the measurement modes. The measurement modes may include one or more of: open circuit potential (OCP) measurement, amperometry measurement, and electrochemical impedance spectroscopy (EIS) measurement.

The measurement circuitry 120 may include a signal generator 122 that generates a signal for providing to a controller 130 selected electrode(s).

The implant device 100 may further include a transmitter/receiver antenna 160 configured to transmit measurements (e.g., in a measurement report) to a patient's device or a clinician's device in order to report detection of infection, bone growth (or lack thereof), etc. The transmitter/receiver antenna 160 may further be configured to receive an instruction(s) from the patient's device of clinician's device, where the instruction(s) include directions on a measurement mode for the implant device to perform or a level/duration of electrical stimulation for the implant device to perform.

Open circuit potential (OCP) involves the passive measurement of the free potential of an electrochemical system when no current is flowing between the reference and working electrodes. A common application of OCP is pH sensing, where potential decreases as pH increases due to the decreasing concentration of hydrogen ions. Additionally, ions such as protons/hydroxonium, sodium, potassium, calcium, and chloride can be detected using potentiometric measurements. For example, the cations sodium and potassium can be measured on ion-selective electrodes when the mode of the potentiostat is potentiometric or open-circuit potential. For example, the measurement circuitry 120 of the implant device 100 may measure the free potential through a first pair of electrodes (of the electrode(s) 110) while the controller 130 of the implant device 100 does not apply current between the first pair of electrodes.

Amperometry, including chronoamperometry (CA), are active techniques that consist of applying a voltage step and measuring the resulting current response at the working electrode. Amperometric techniques are used for detection of small molecules through electrochemical reduction. One application of CA is dissolved oxygen (DO) sensing, where the measured current response increases as the percentage of dissolved oxygen decreases. Another application is found in blood glucose test-strips and newer continuous glucose monitors, which rely on the CA technique to quantify the concentration of glucose in the blood or interstitial fluid. For example, the controller 130 of the implant device 100 may apply power to an electrode in a second pair of electrodes while the measurement circuitry 120 of the implant device 100 measures current resulting at the other electrode in the second pair of electrodes. In these embodiments, direct current (DC) may be used.

Electrochemical impedance spectroscopy (EIS) is one of the most complex and powerful methods in analytical electrochemistry. EIS involves the application of a sinusoidal (AC) potential across a range of frequencies and measurement of the magnitude and phase angle of the resulting current. An equivalent circuit model of the electrochemical system is often constructed with EIS data and is used to isolate and investigate changes in individual components of the system (e.g. biochemical layers) over time. One application of EIS is medical diagnostics, where the impedance of a cancerous tissue structure may be differentiated from that of healthy soft tissue. For example, the controller 130 may apply an alternating current to one of the electrodes in a third pair of electrodes while the measurement circuitry 120 measures current resulting at the other electrode in the third pair of electrodes.

Infection detection via electrochemical sensing is discussed in further detail below.

Electrochemical sensing methods offer promise for the development of a implantable biofilm sensor because they can provide insights into electrochemical activity of the microenvironment on the surface of the sensor and macroenvironment surrounding the sensor.

OCP, CA, and EIS may be of interest for measuring changes in the biofilm physiology analogous to pH, dissolved oxygen, and impedance, respectively. It is believed that metabolic waste products, such as lactic acid and citric acid, accumulate within the EPS as biofilm grows, which causes the biofilm to become more acidic (lowerpH) over time. Similarly, metabolic oxygen is believed to be consumed as biofilm develops, and the decrease in oxygen could be most drastic at the greatest depth (i.e. biofilm-electrode interface). Lastly, the biofilm impedance (i.e. resistance) is believed to increase due to an increasing obstruction in current flow across the EPS as the biofilm thickens over time.

Infection treatment via electrical stimulation is discussed in further detail below.

Electrical stimulation can be precisely controlled and steered, by a controller in the implant device, to target only the biofilm on the surface of the implant without harming the surrounding tissue.

For example, 0.1 mA of DC current applied for 21 days may prevent signs of clinical infection around percutaneous pins implanted in a tibias. Electrical stimulation may enhance the efficacy of antibiotics and greatly reduce the concentration needed to eradicate biofilms. For example, a 2-log (~99%) reduction in E. coli biofilms adhered to dental implants may be achieved using a continuous current of 0-10 mA for 15 minutes. This short duration of treatment could enable the stimulation to be used during in-office doctor procedures.

Infection detection & treatment via smart implant system is discussed in further detail below.

The smart implant system described herein may be capable of sensing and eradicating biofilm growth thereby enabling early detection and treatment of implant-related infections such as PJI and SSI.

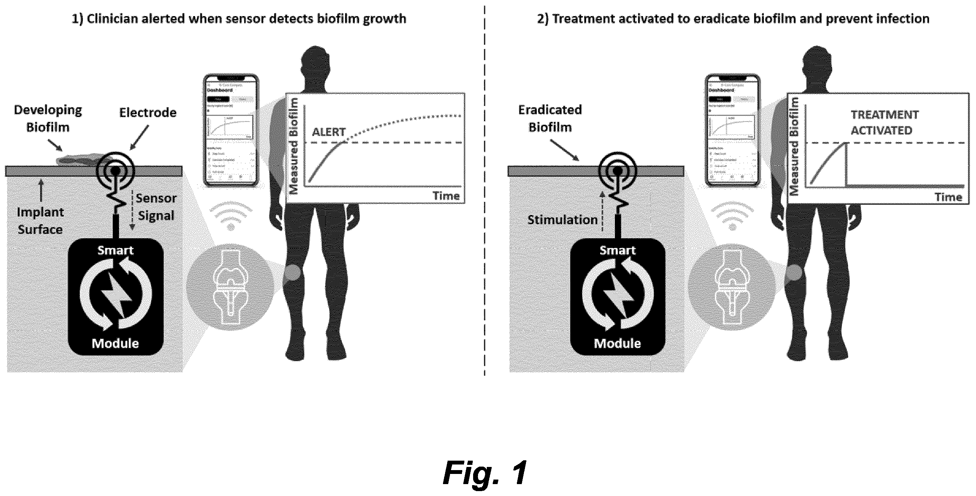

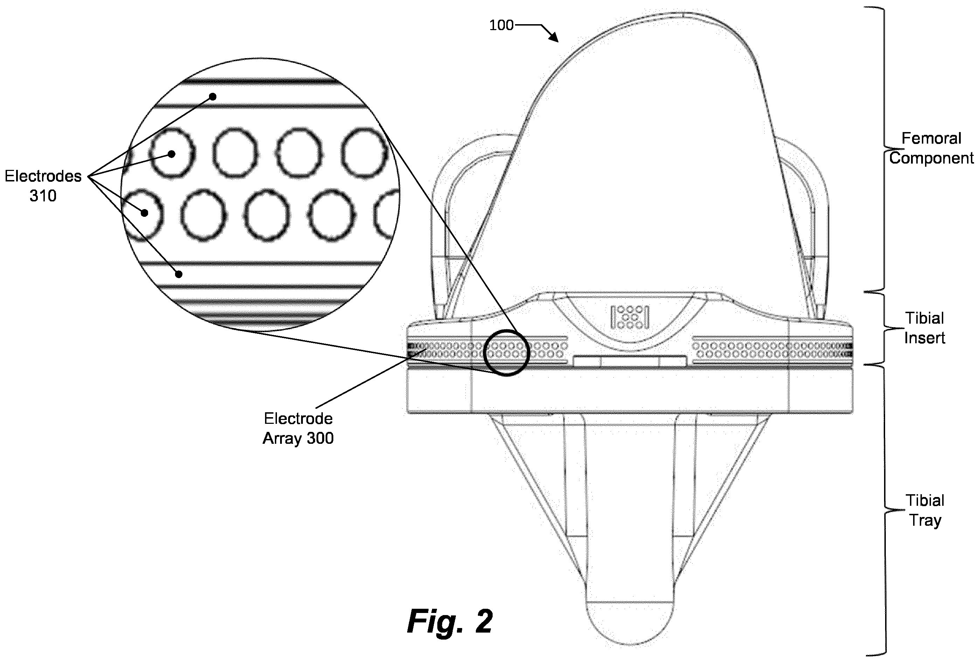

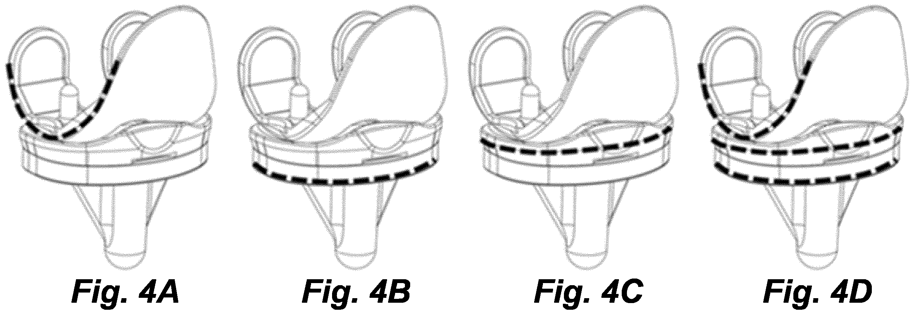

Figures 1 illustrates an example of a smart implant system configured to alert a clinician of biofilm growth and treat the biofilm to help prevent infection, according to some embodiments of the present disclosure. Figure 2 illustrates an example of the smart implant system for total knee arthroplasty (TKA), according to some embodiments of the present disclosure. Figure 4A-D illustrates different locations where sets of electrodes in an array may be located on the smart implant system for TKA, according to some embodiments of the present disclosure. The different locations of the sets of electrodes in an array are indicated with the dark thick dashed line in the Figures 4A-4D.

One embodiment of this system includes a TKA implant component (e.g. tibial insert) with electrodes integrated along different parts of its surface. In this concept, the array of electrodes is controlled by an embedded smart module (also referred herein as a controller (e.g., controller 130 of Figure 27)) that functions as an electrochemical sensing and stimulation system. The location and surface topology of these electrodes may be optimized to bias biofilm formation on the electrodes prior to other areas of the implant. As the patient recovers from surgery, measurement data (captured by the measurement circuitry via the electrodes) is transmitted (via a transmitter/receiver) to a patient's device (e.g., patient's laptop, smartphone, etc.) and forwarded to a clinical for review. If the system detects early stages of biofilm formation, the clinical is alerted to prompt closer follow-up, early diagnosis, and/or treatment. For example, the measurements captured by the measurement circuitry may be stored locally on the implant device or a device external to the patient and mapped over time to detect a threshold change or a change over time that indicates a possibility of infection.

With this information, the clinician may opt to remotely activate a noninvasive stimulation treatment to eradicate the biofilm. In combination with a standard antibiotic regimen, this treatment may prevent the development of an infection and eliminate the need for surgical intervention. Artificial intelligence may be added to this system to form a closed-loop system, which autonomously and continuously identifies and eradicates early-stage biofilms prior to the onset of infection symptoms.

It should be noted that while the present disclosure describes smart implant embodiments designed to detect and treat infection, the present disclosure is not limited thereto but rather also includes other clinical applications such as oncology and pain management. Additionally, smart implant refers to integration of electronics with any implant used for musculoskeletal surgery, including spine implants (e.g. interbody spacer, rod, screw), joint implants (e.g. total hip, total knee, total shoulder), and trauma implants (e.g. plates, nails, screws, external fixators).

A smart musculoskeletal implant is an implantable medical device with control and communication capabilities designed to promote healing, prevent complications, and/or monitor surgical outcomes in patients with musculoskeletal disorders. An embodiment of a smart musculoskeletal implant (i.e. smart implant) includes a control and communication module that is assembled to, integrated with, or part of a traditional musculoskeletal implant, which is further defined below.

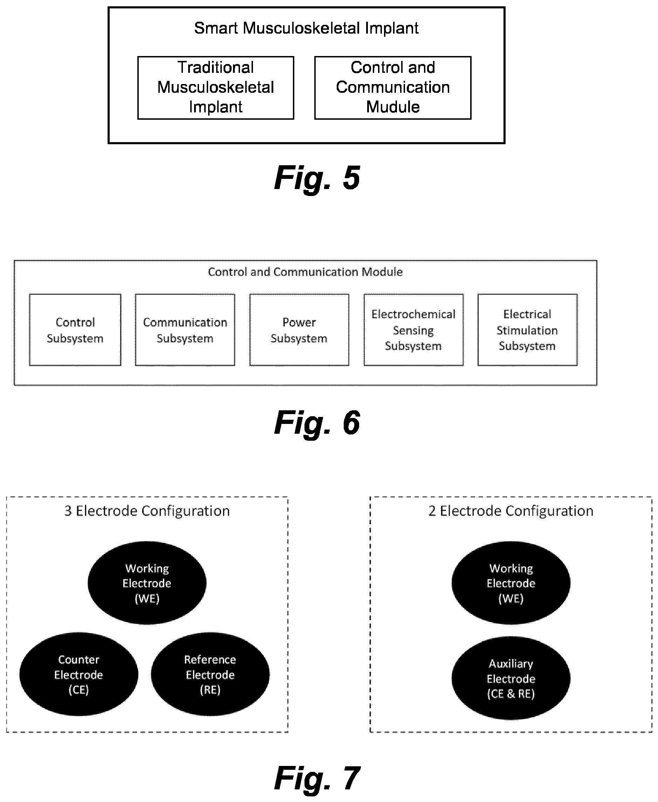

Figure 5 illustrates an embodiment of a smart musculoskeletal implant that includes a control and communication module that is assembled to, integrated with, or part of a traditional musculoskeletal implant, according to some embodiments of the present disclosure.

Traditional musculoskeletal implants include any implant designed for musculoskeletal surgery, including spine, trauma, and joint procedures, which can be made 'smart' by the addition of an electronics module (e.g., a control and communication module).

Figure 6 illustrates an example of a control and communication module, according to some embodiments of the present disclosure.

The control and communication module may be the elements which makes the system 'smart'. The control and communication module, at minimum, includes a control subsystem, communication subsystem, and power subsystem. In some optional embodiments, the smart implant further includes an electrochemical sensing subsystem, electrical stimulation subsystem, or both.

Some embodiments of the smart implant may have a primary function of stability and restoration of musculoskeletal structures and a secondary function to sense and act upon its surroundings with electrochemical means.

Infections may be produced by pathogens, including bacteria, which thrive in biofilms on implant surfaces and which effect the microenvironment of the implant surfaces and macroenvironment of the implant surroundings, including the pH level, oxygen concentration, and electrical impedance. Additionally, such biofilms and the pathogens housed within may be eradicated with low-levels of electrical stimulation.

Generally, an electrochemical sensing system may include a dedicated electrochemical cell (i.e. set of 2 or 3 electrodes) for each sensor measurement and analyte (e.g. pH and dissolved oxygen). The working electrode may be functionalized to enable a sensitive and specific reaction with the target analyte.

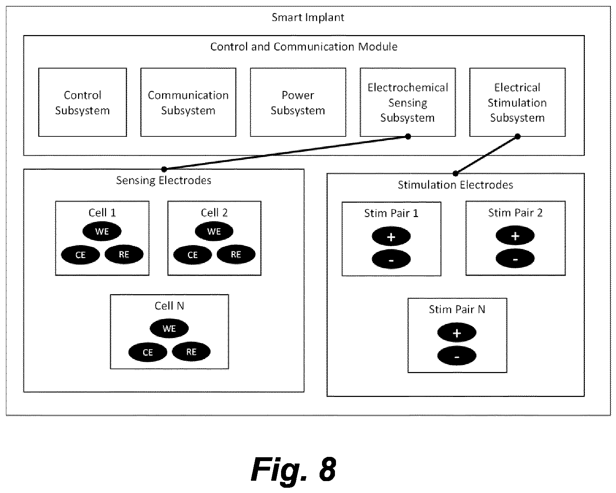

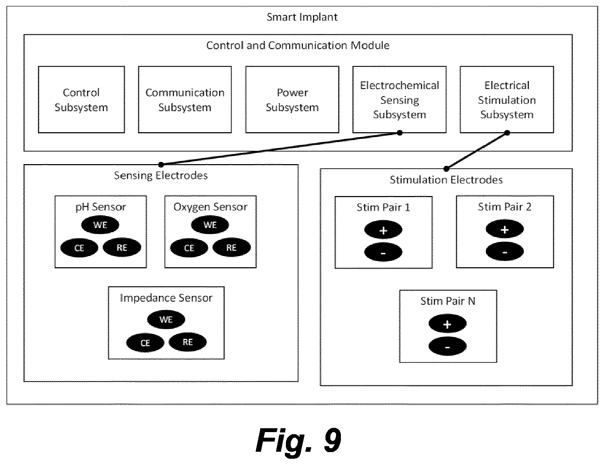

Figure 7 illustrates a three-electrode and two-electrode configuration, according to some embodiments of the present disclosure. Figures 8-9 illustrates examples of a smart implant with three-electrode configurations with functionalized working electrodes, according to some embodiments of the present disclosure.

Electrochemical measurement of the oxygen concentration, the pH level, and impedance may include a dedicated set of electrodes for each measurement. Further, electrical stimulation may be facilitated through a separate, dedicated sets of anode (+) and cathode (-) electrode pairs. As shown in Figures 8-9, a 3-electrode configuration would require 9 electrodes to perform all 3 electrochemical measurements and one or more additional electrode pairs for stimulation.

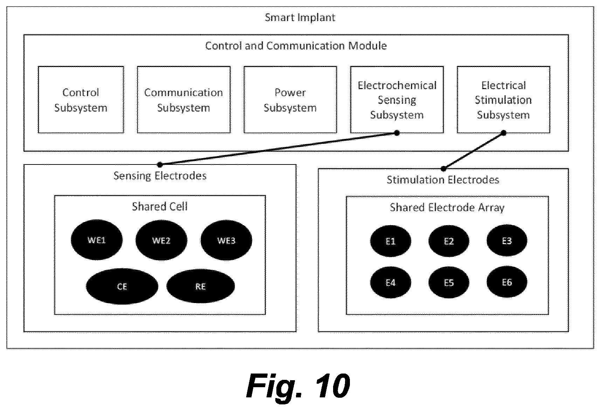

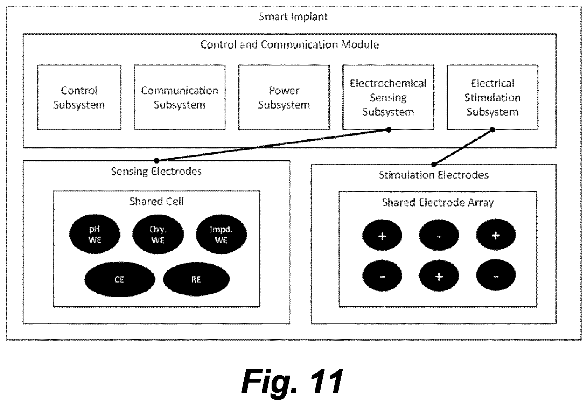

Figures 10-11 illustrate examples of a smart implant with working electrodes in a shared cell and separate electrode arrays for measuring/sensing and stimulation, according to some embodiments of the present disclosure.

To reduce the space requirements of the system and overall number of electrodes, the counter and reference electrodes may be shared by different working electrodes, however a unique working electrode (i.e. sensing electrode) is generally required for each analyte. Therefore, electrochemical measurement of the oxygen concentration, the pH level, and impedance may need a dedicated working electrode (i.e. sense electrode) for each measurement. Additionally, a shared electrode array could be utilized such that each electrode could serve as the anode or cathode on-demand via programming (i.e. multiplexing) depending on the scenario of use. This electrode configuration enables the stimulation to be precisely controlled and steered.

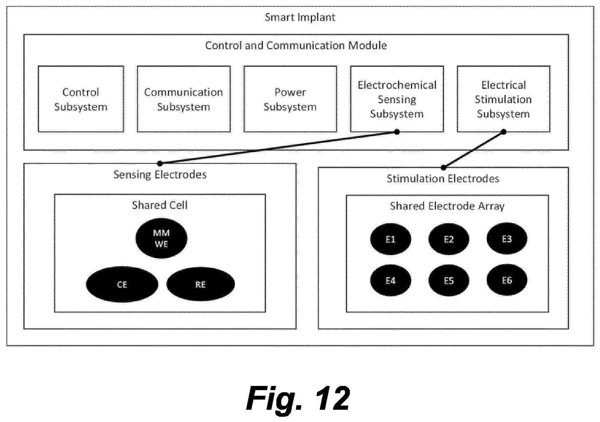

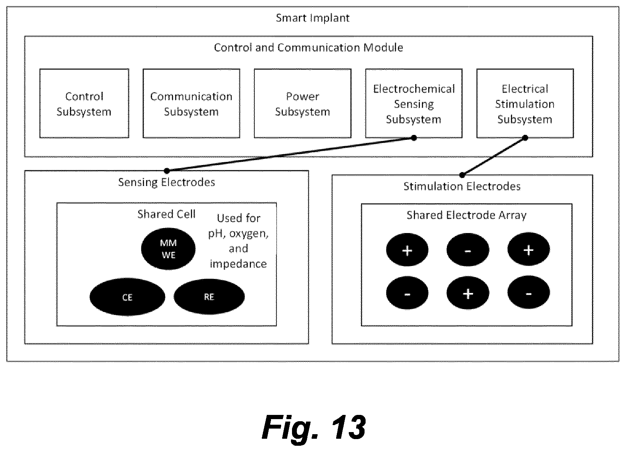

Figures 12-13 illustrate examples of a smart implant with a multimodal working electrode and separate electrode arrays for measuring/sensing and stimulation, according to some embodiments of the present disclosure.

In these examples, the smart implant includes a sensing electrode array and another electrode array that is used as a stimulation electrode array. The another electrode array may be spaced apart on the implant device from the sensing electrode array and may include another set of electrodes that are physically separate and electrically isolated from the set of sensing electrodes in the sensing electrode array. In some of these embodiments, the controller controls the multiplexer to selectively connect different pairs of electrodes in the another array over time to sequentially perform the measurement modes and/or perform electrical stimulation.

In some preferred embodiments, exemplified by Figures 12-13, oxygen concentration, pH, and the impedance/dielectric properties are measured on a single, multimodal working electrode. It is possible to measure multiple analytes at a generic, multimodal working electrode (MMWE) and therefore reduce the size and complexity of the electrode array. For example, in Figure 12, a multimodal working electrode (MM WE) may be configured to measure a plurality of oxygen concentration, pH, and the impedance/dielectric properties. Additionally, each electrode E1-6 in the stimulation electrode array may be used to generate an electric field between pairs of the electrodes (at different times or at once) to electrically stimulate the tissue around the implant.

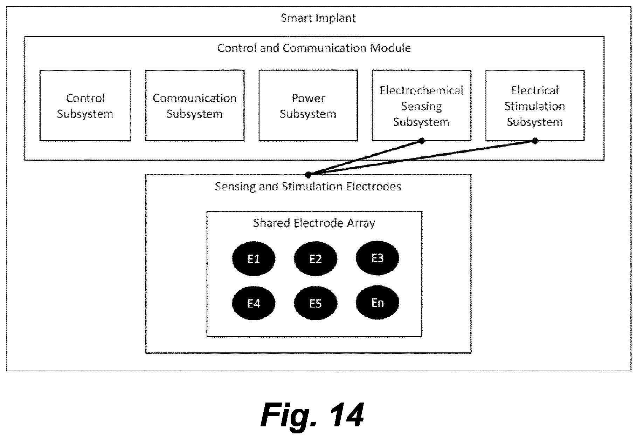

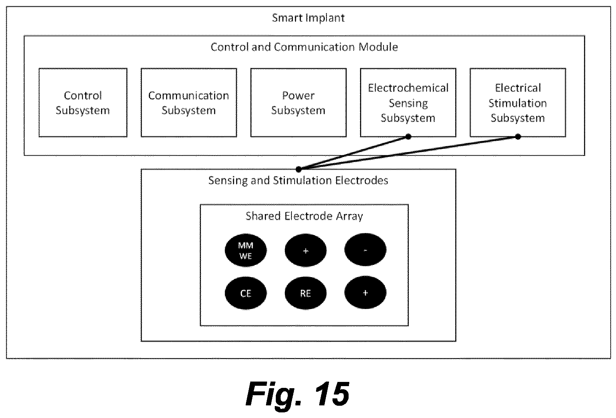

Figures 14-15 illustrate a smart implant with a plurality of multimodal working electrodes and the same electrode arrays for measuring/sensing and stimulation, according to some embodiments of the present disclosure.

In some preferred embodiment, exemplified by Figures 14-15, a multimodal electrode array is utilized by both electrochemical sensing and stimulation subsystems and any electrode in the array can be specified for any function (e.g. WE, RE, CE, anode, cathode) via multiplexing and programming (via the controller and multiplexer of the implant device). This may further reduce the size and complexity of the sensing and stimulation systems to a minimum, which may be preferred for the miniaturization requirements of smart implant applications.

Additionally, the function of any electrode in the electrode array can vary with time. For example, three different electrochemical measurements may be captured from a single multimodal working electrode with adjacent electrodes acting as counter and reference electrodes. Then at a later time point, the role of each electrode could alternate to enable multiple electrochemical measurements to be performed at every electrode in the array. For example, a first electrode in a set of three electrodes may act as the working electrode while a second electrode acts as the counter electrode. Then at a later time, the second electrode may act as a working electrode and the first electrode may act as the counter electrode. Similarly, each electrode in the array could be alternated as an anode or cathode to enable electrical stimulation at every electrode in the array. This function switching enablement is further discussed below.

Sensing sequence is discussed in further detail below.

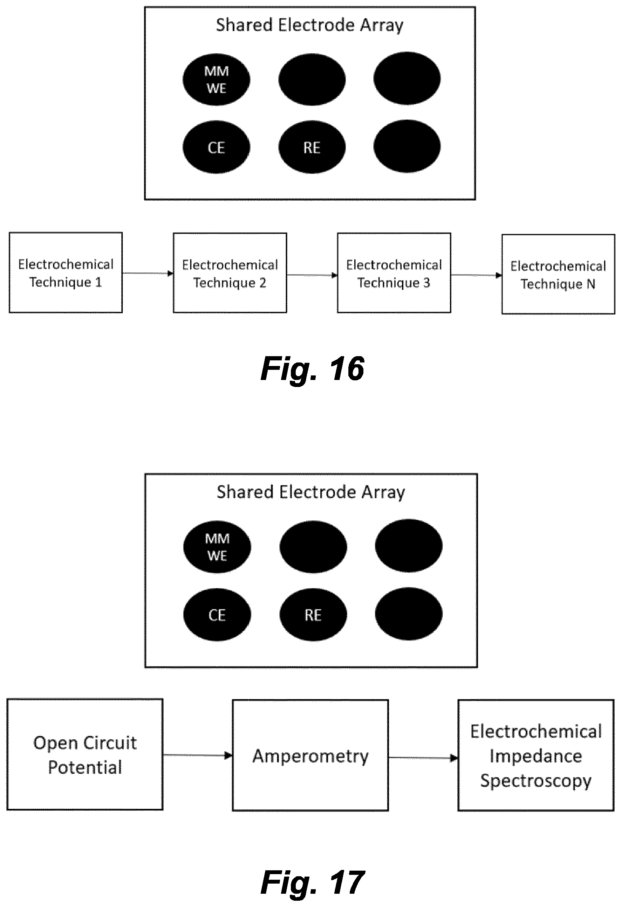

Figure 16 illustrates an electrode array that is controlled to perform measurement modes sequentially, according to some embodiments of the present disclosure.

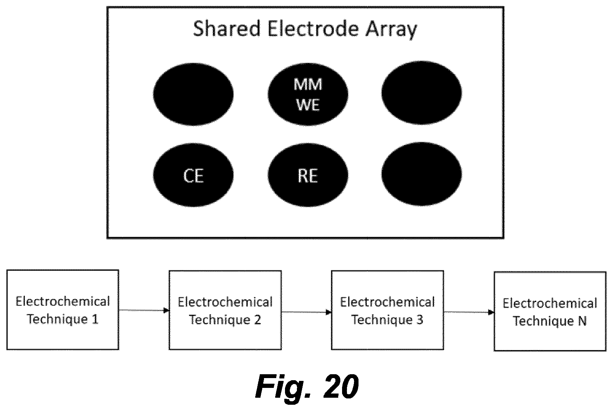

In some embodiments, the multimodal electrode array includes three or more electrodes whose function can vary with time. For example, one electrode could be designated as the working electrode, another as the counter electrode, and a third as the reference electrode. Then, at another time, the electrodes functions could switch (e.g., from acting as a working electrode to a reference electrode) or at least one electrode could be switched (e.g., a new working electrode is selected by the multiplexer via the controller and the previously used working electrode is unselected by the controller). Figure 20 illustrates an example of the controller controlling a multiplexer to select a new working electrode to perform a series of electrochemical techniques, according to some embodiments of the present disclosure. In this example, after a first working electrode performs one or more techniques a second electrode in the electrode array is selected by the controller to perform a series of techniques.

In order to facilitate measurement of different analytes (i.e., performance of different measurement modes) with the same working electrode, various electrochemical techniques may be performed sequentially. For example, Technique 1 could be executed during a first time window by a first pair of electrodes in the electrode array (e.g., the controller controls the multiplexer to select a first electrode in the pair of electrodes to operate as a working electrode and a second electrode in the pair to operate as a counter electrode), followed by Technique 2 during a second time window by a second pair of electrodes in the electrode array, Technique 3 during a third time window by a third pair of electrodes in the electrode array, and so on. When performed sequentially, the different time windows are separate time windows that do not overlap in time with each other. Additionally, the first, second, third pairs of electrodes may be the same pair or a different pair of electrodes in the electrode array.

In some embodiments, during a non-overlapping time window relative to the first time window the controller controls the multiplexer to select the first electrode to operate as the counter electrode and selects a third electrode, that is different than the second electrode that operated as a counter electrode, to operate as the working electrode.

Pauses may be incorporated between techniques (i.e., measurement modes and/or electrical stimulations) for various reasons, including to enable distribution and balance of charges. For example, between a sequence of two of the techniques, the controller is configured to delay a time period during which a controller of the implant device applies (e.g., via the measurement circuitry 120) no current or a reverse biased current to the pair of electrodes used during a prior completed (or one of the previous) techniques. The reverse biased current may be a reverse bias of the current that was applied during the prior completed (or one of the previous) technique.

Figure 17 illustrates an example of an electrode array that performs open circuit potential, amperometry, and electrochemical impedance spectroscopy in series, according to some embodiments of the present disclosure.

In the example of electrochemical sensing for detection of biofilm and infection, the electrochemical measurement sequence might include open circuit potentiometry (i.e. pH), followed by amperometry (i.e. oxygen), followed by electrochemical impedance spectroscopy (i.e. biofilm impedance). Each technique may be performed by the same two or three electrodes, each techniques may be performed by a different two or three electrodes, or some of the electrodes may be reused from technique to technique.

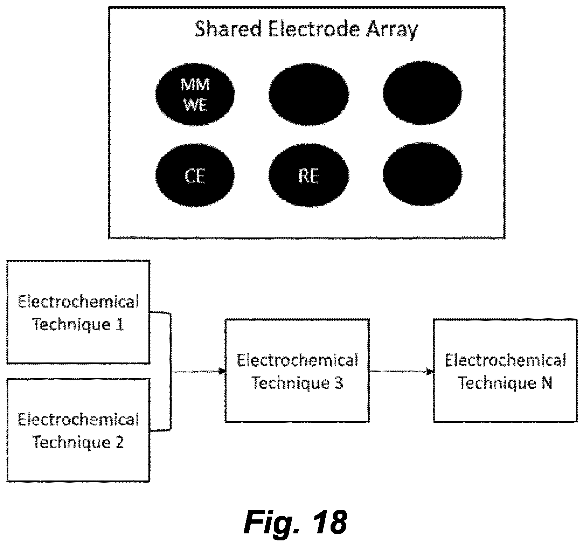

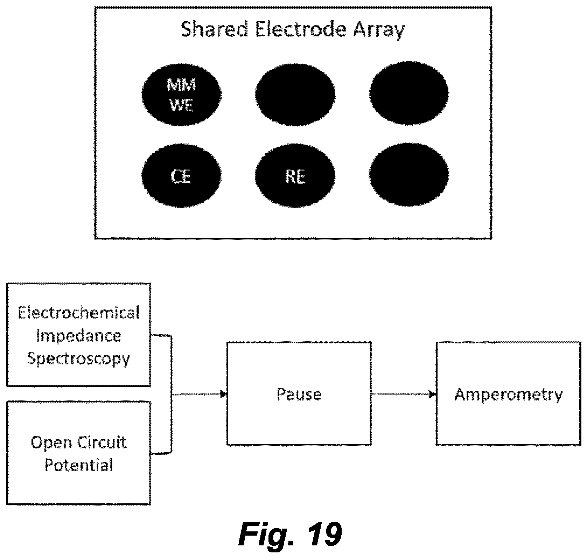

Figure 18 illustrates an example of an electrode array that performs two electrochemical techniques in parallel, according to some embodiments of the present disclosure. Figure 19 illustrates an example of an electrode array that performs EIS and OCP in parallel, according to some embodiments of the present disclosure.

All of the techniques in a measurement process do not necessarily need to be in series. For example, Technique 1 and Technique 2 could be executed in parallel followed by Technique 3. In an infection detection example, EIS and OCP may be measured in parallel, followed by a pause, and then followed by amperometry.

To be considered in parallel, the techniques may be performed in at least partially overlapping time windows. For example, a first measurement mode of the plurality of measurement modes may be performed during an at partially overlapping time window while a second measurement mode of the at least two measurement modes is performed, and the pair of electrodes that is used in the first measurement mode is different than the pair of electrodes that is used in the second measurement mode. However, the pair of electrodes may share at least one electrode (e.g., a shared counter electrode).

However, it should be noted that while two techniques are performed in parallel, in Figures 18 and 19, more than two electrochemical techniques can be performed in parallel.

The system also enables each electrode to serve as the working electrode. The previously detailed sequential measurements could be performed on a different electrode that is designated as the working electrode. This is discussed in more detail below.

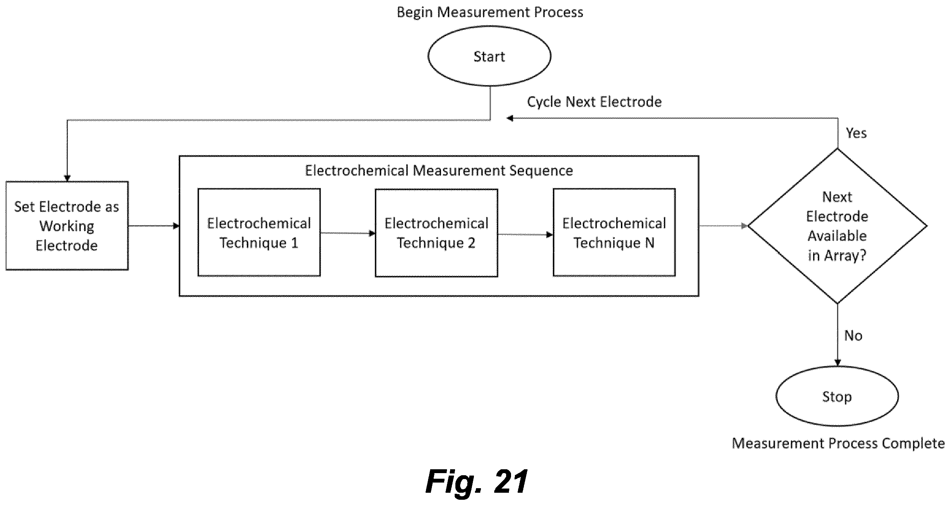

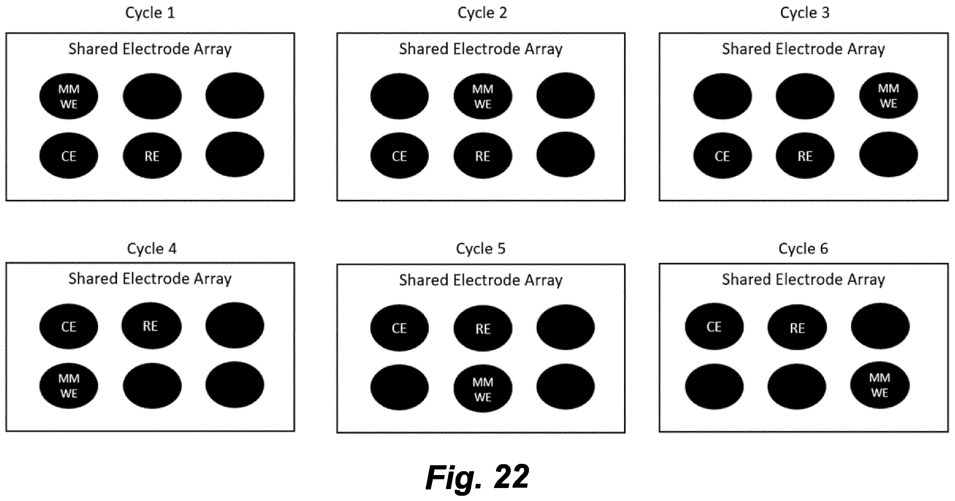

Figure 21 illustrates a flowchart of a measurement process where electrodes in an electrode array are cycled through, according to some embodiments of the present disclosure. Figure 22 illustrates electrodes in an electrode array be cycled through during the measurement process of Figure 21, according to some embodiments of the present disclosure.

By cycling through the electrochemical measurement sequence at each electrode, a map of the electrochemical environment surrounding the electrode array can be constructed. The map may be generated by the implant device (e.g., via the controller) and/or the measurements may be reported in a measurement report to an external device (e.g., patients phone or clinician device) for generation at the external device. This enables spatial understanding of where biofilm or infection may be present versus not present.

In some of these embodiments, the controller generates a measurement report indicating results of the performed measurement modes, and controls transmission of the report to a device that is external to the body of the patient.

In the examples of Figures 21-22, the measurement process is designed to determine whether there is an infection on or around the area of the implant. The measurement process could be executed automatically and/or repetitively by the smart implant system at time intervals electronically settable in memory of the implant device. The memory may be physically separate but electrically connected to the controller of the implant device. Alternatively, the memory of the implant device could be part of the controller. For example once per day or once per week. However, shorter or longer time intervals may be used and the length of the time intervals may vary over time depending on the measurements (e.g., if infection is detected) or a time since implantation of the implant device in the patient. Shorter intervals may be desired in the post-acute period to enable early detection of infection when the patient is must susceptible. For example, the implant may execute the measurement process every two hours for the first 14 days following surgery. Longer intervals may be desired later in the recovery period to conserve battery life or avoid patient compliance issues with wirelessly powering the device. For example, from day 14 up to day 90, the implant may execute the measurement process once per day or once per week. After 90 days, the incidence of infection decreases significantly and measurements may not need to be collected automatically. In circumstances where the patient is at high risk for infection after 90 days, such as in the case of dental work, other surgeries, or frequent urinary tract infections, the measurement process and protocol could be reinitiated by the patient or clinician. In addition to automatic execution of the measurement process, measurements could also be manually initiated at any time by the patient or clinician using the connectivity system.

The measurement process may begin by a controller of the implant device determining or controlling the multiplexer to select a set of electrodes that are capable of functioning as working electrodes (e.g., the shared electrode array of Figure 22). For example, the controller may control the multiplexer to sequentially select each electrode in the set of electrodes in the array, during different time windows, to operate as a working electrode while the controller sequentially performs at least two measurement modes.

Then the controller may select (via a multiplexer) a pair (or more) of electrodes in the set of electrodes to perform an electrochemical measurement sequence with. The electrochemical measurement sequence may include one measurement mode or a plurality of measurement modes (e.g., electrochemical technique 1-N). The controller may use a measurement circuitry, connected through a multiplexer that interconnects different pairs of electrodes in the array to the measurement circuitry, to measure the signals received by the electrodes. After the pair of electrodes in the set of electrodes has performed the electrochemical measurement sequence (Cycle 1), the controller may determine if there is another pair (or more) of electrodes in the array that are available (or have not performed the measurement sequence) to perform a Cycle 2. If there are not electrodes available, then the measurement process ends. If there are electrodes available, then the controller starts the measurement process from the beginning (Cycle 2) and selects a different electrode pair (or more) to perform the electrochemical measurement sequence. This may continue until every electrode in the electrode array has performed the electrochemical measurement sequence as a working electrode, may continue until a threshold number of electrodes in the electrode array has performed the electrochemical measurement sequence as a working electrode, may continue for a predefined time period.

A cleaning routine is discussed in further detail below.

The smart implant system may be implanted in-situ for several years and the sensors may have several months of operation and occasional operation after that. Therefore it may be necessary to occasionally replenish or clean the working (i.e. sensing) electrodes. Cleaning may be necessary because of biofouling or buildup of biological material on the surface of the electrode which causes reduction in sensitivity and increase in surface impedance. A series of potential and/or current steps or bursts may result in a 'cleaning routine' when the electrodes are considered fowled, corroded, or encapsulated. The electrodes may be determined to be in need of cleaning based on the measurements taken by the electrodes (or near the electrodes) indicating fowling, corrosion, or encapsulation over time (e.g., through impedance measurements that indicate an abnormality).

Stimulation sequence is discussed in further detail below.

Electrical stimulation can be used as an effective treatment for eradicating bacteria and biofilms. Any of the electrodes within the multimodal electrode array can be designated as anodes or cathodes for the purposes of electrical stimulation. Control over which electrodes serve as the anode and cathode may enable steering of the electric field over the adjacent volume to direct the treatment energy. Sequential switching of the anode and cathode configuration may ensure that all electrode surfaces are sufficiently treated. This may be important for stimulation programs where the treatment is more effective at the cathode vs the anode or vise versa.

Regarding the electrical stimulation of the working electrodes, a controller may be operative to control electrical stimulation of specific working electrodes, or all the working electrodes of an electrode array, by supplying direct voltage from an energy storage device within the smart implant to be at a level which at least reduces formation of a biofilm on at least part of the implant component while implanted in the patient. For example, the controller may control supply of a direct voltage to one pair of the electrodes in the array and controls supply of an alternating current swept across a range of frequencies to another pair of the electrodes in the array.

In another example, the implant circuitry may provide a voltage differential between a working electrode(s) and a counter electrode to cause electric field(s) to extend therebetween, and controls the level of current density therebetween to at least reduce formation of a biofilm and/or to at least partially eradicate a biofilm on at least part of the implant component while implanted in the patient. The counter electrode may be the counter electrode in the same electrode array or a counter electrode of a different electrode array than the one in which the working electrode(s) are being stimulated at. Alternatively, there may be a counter electrode that is not a part of an electrode array that is used while a working electrode(s) is stimulated.

The stimulation sequence may be used independent of the measurement process. For example, the patient or clinician may only want to leverage the infection treatment functions as needed and not use the other smart implant functions. In these instances, the clinician has a non-invasive treatment option that can be activated on-demand. If the patient reports symptoms of infection, the clinician could remotely activate a treatment or the patient could visit the clinic for an in-office treatment. In either case, the implant may act as an on-demand, in situ delivery system for species-agnostic infection treatment.

The stimulation sequence can also be used in conjunction with the measurement process to enable a sensing and treatment control loop. For example, the measurements from the first 14 days post-surgery may indicate growth of a biofilm that has not yet presented infection symptoms. The clinician could choose to more closely monitor the patient for development of symptoms before administering treatment, or a treatment could be activated with the smart implant as preventative measure.

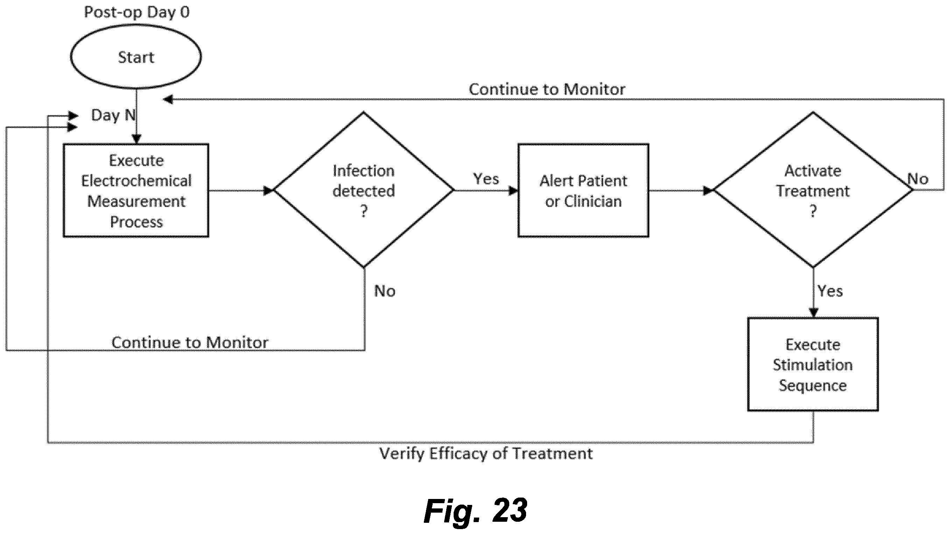

Figure 23 illustrates a flowchart of a stimulation sequence used in conjunction with a measurement sequence, according to some embodiments of the present disclosure. It should be noted that while each step in the process is described to be performed by the implant device (e.g., a controller in communication with other components of the implant device), some steps may be performed by, or in conjunction with, an external device.

After the implant is implanted in the patient or a number of days (Day N) after, the implant device may execute electrochemical measurement process (including at least one measurement mode). For example, the measurement mode(s) may be performed by the controller responsive to expiration of a time period measured since implantation of the implant device within the body of the patient.

Based on the measurements, the implant device (e.g., the controller in the implant device) may determine whether an infection is detected. If an infection is not detected, then the implant device may start the process over again. If an infection is detected, then the implant device may alert the patient (e.g., by transmitting a message to a device of the patient) or the clinician (e.g., by transmitting a message to a clinician device directly, via a patient device, or via an external device that is used to transmit/receive message to/from the clinician). Then the implant device may determine to activate treatment (i.e., electrical stimulation). Alternatively, the clinician may determine to activate treatment and transmit (through a clinician device) instructions to the implant device to activate treatment.

Optionally, the implant device may determine a level (i.e., the voltage/current) at which to generate the electrical stimulation.

If it is determined to activate treatment, then the implant device executes a stimulation sequence (e.g., generating an electric field with low current for a duration of time, generating a plurality of potential and/or current bursts, etc.). After executing the stimulation sequence, the process may start over in order to verify efficacy of treatment or if further treatment is desired. If it is determined not to activate treatment, then the process starts over.

Osteogenic applications are discussed in further detail below.

Osteogenic (i.e. bone growth) complications are a factor in the majority of musculoskeletal surgeries. Bone growth issues are a significant concern in spine fusion, long-bone trauma, and cementless joint arthroplasty, especially if the patient is considered high-risk (e.g. smoking history, obesity, osteoporosis, prior surgeries, diabetes, etc.).

Implant devices may provide therapeutic benefits through direct current electrical stimulation (DCES) in an accepted form factor and workflow. The addition of DCES technology into static and expandable interbody spacers, including next generation 3D printed expendables, could further enhance the clinical effectiveness of these implants. The smart implants could be offered in an open-loop configuration where a constant dose is delivered for approximately six months following surgery. With the addition of the previously described electrodes, the implant could also monitor the effects of stimulation on the progression of fusion and enhance the dose if needed. This feedback loop could also be controlled autonomously by the implant, allowing the implant to adapt the therapy to the needs of each individual patient.

Some smart implants may include a conductive graft scaffold. These smart implants may accelerate bone formation across a disc space with the conductive graft scaffold. The conductive graft scaffold may act as a three-dimensional (3D) electrode to deliver and distribute electrical bone growth stimulation through the implant graft window. The scaffold may be advantageous over electrodes integrated into the interbody implant surfaces because the electric field generated by the conductive graft scaffold may be more uniform across and targeted to the graft material. An elastomeric structure may provide the ability to collapse to a compact shape during insertion and expand along with the interbody during an implant expansion operation. Generally, elastomeric materials are nonconductive, so the graphene coating can render the elastomeric structure as conductive.

A portion of the smart implant may include an anodic housing that is configured to create an anode charge and the graft scaffold may be configured to create a cathode charge. At a certain voltage and/or frequency, the electric field formed between the anodic housing and the graft scaffold may accelerate bone formation across the disc space.

As with the infection applications previously described in this disclosure, there may be a need for a multimodal electrode array that can be utilized for both electrochemical sensing and electrical stimulation of bone growth. It should be noted that embodiments described in the content of infection applications may also apply to the bone growth applications herein. Additional details about the sensing and stimulation system for osteogenic applications are presented below.

Electrochemical monitoring of bone healing is described in further detail below.

Fusion monitoring can be facilitated by measuring changes in impedance of the tissue surrounding the implants over time. The impedance measurement system leverages a set of electrodes and potentiostat subsystem (which may be included in the measurement circuitry). Each distinct type of tissue has unique dielectric properties which can be measured and correlated with EIS techniques. By measuring the impedance of the adjacent graft material over time, the system will enable the clinician to assess the change in tissue type from aggregate graft material to cortical bone. Additionally, the impedance values can also be correlated with bone mineral density measurements to not only report the formation of bone, but more specifically the bone mineral density of that bone tissue. With the addition of electrical impedance tomography (EIT) post-processing algorithms, this system can also construct a three-dimension (3D) visualization of the tissue location and composition surrounding the implant. In some embodiments, the controller performs an EIS measurement and the controller is configured to perform electrical impedance tomography (EIT) using the measurements taken during the EIS measurement to construct a three-dimensional (3D) model of tissue location and characteristics adjacent the set of electrodes. In some of these embodiments, the implant device may generate or otherwise construct the 3D model. In other embodiments, the implant device may report the measurements to a device external to the patient for construction of the 3D model at the external device.

The proposed smart implant system may provide clinicians a robust tool for clinical decision making, including healing assessments, follow-up protocols, and treatment plans. In addition, the data collected by this system could be used to train diagnostic algorithms that are sufficiently accurate and reliable to provide clinical diagnosis of delayed fusion, non-fusion, and mechanical breakage. These diagnostic algorithms may be coupled with the bone growth stimulation system (discussed more below) to form a closed-loop fusion enhancement system which automatically steers the stimulation and adjusts the dosage without intervention from the clinician or patient.

Electrical stimulation of bone growth is discussed in further detail below.

Bone growth acceleration is enabled by electrical stimulation, which exploits the natural bone modeling process and enhances osteogenic activity. Cathodic-biased current pulses are delivered to the adjacent bone graft tissues using the same set of electrodes that were described for sensing/measuring. Current-controlled pulses are generated by a the controller via the measurement circuitry (e.g., via a signal generator that may include a pulse generator or a potentiostat IC). The stimulation current may be biased cathodic because cathodic current has been shown to be more effective at inducing osteogenic activity, such as Alkaline Phosphatase, as compared to anodic current. For example, the implant device may perform an EIS measurement, and based on the measurement the implant device may control a cathodic biased electrical stimulation of different pairs of electrodes in an array responsive to measurements taken during the EIS measurement indicative of amount of bone growth.

Biphasic pulses may be employed to balance charges between the designated anode and cathode electrodes. In an example, the maximum voltage may be set to 1.5V or less to avoid electrolysis, and the pulse frequency ranges between 0.5 Hz and 100 Hz with a pulse duration of less than 1 millisecond and amplitude less than 1mA. The implant device and accompanying software may enable the clinician to program the stimulation treatment, much like programming of a spinal cord stimulator. Programming may enable the clinician to set which electrodes are activated (or may provide instructions on how the controller of the implant device is to select which electrodes to activate) along with the treatment current amplitude, frequency, pulse profile, pulse duration, and total treatment duration. One example of a program may include a biphasic square-wave with a frequency of 10 Hz, pulse duration of 0.5 milliseconds, and amplitude of 100 µA. This program may then be prescribed within a treatment protocol, which could consist of 1 hour of treatment per week for 24 weeks. Over time, the clinician-specified treatment programs can be correlated with measured fusion outcomes (measured by the implant device) to assess which programs and protocols are most effective for a particular patient population.

Bone fusion detection & treatment via smart implant system is discussed in further detail below.

Some embodiments of the present disclosure, are discussed with reference to a minimally invasive transforaminal lumbar interbody fusion (MIS TLIF). However, the disclosed embodiments may also apply to any type of musculoskeletal implant.

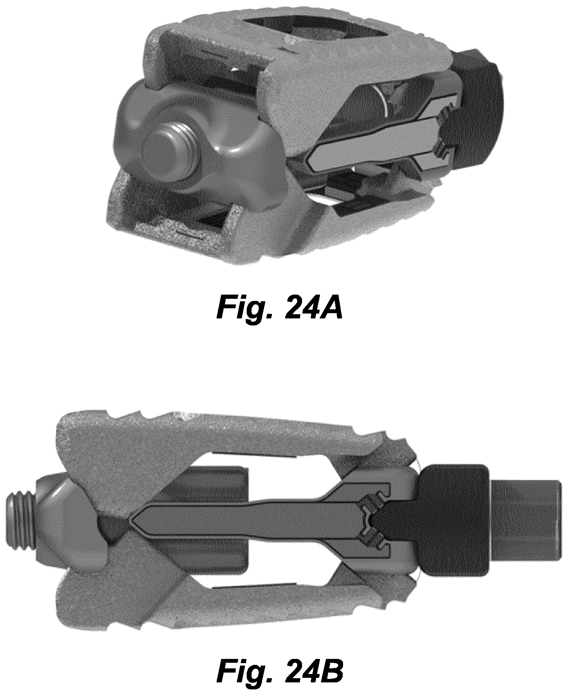

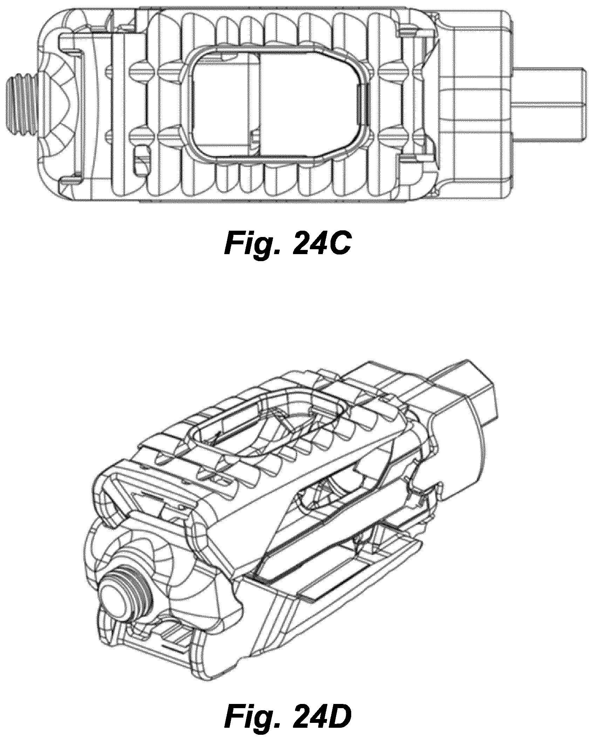

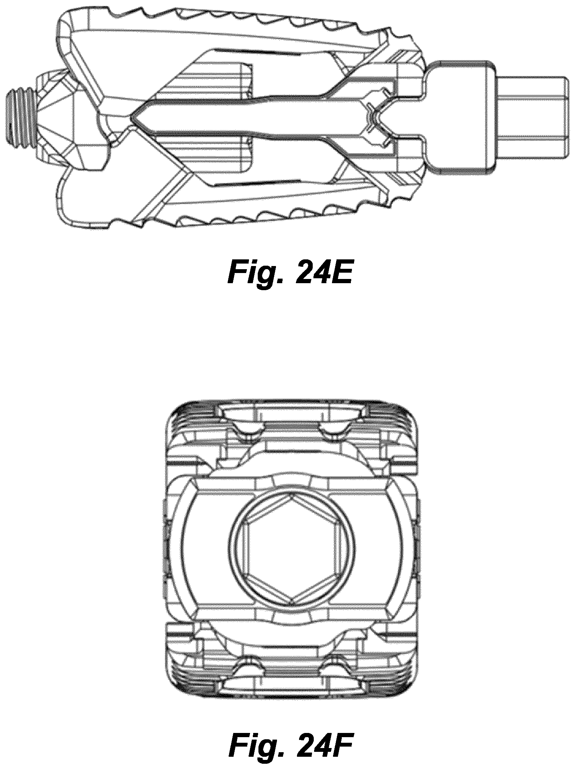

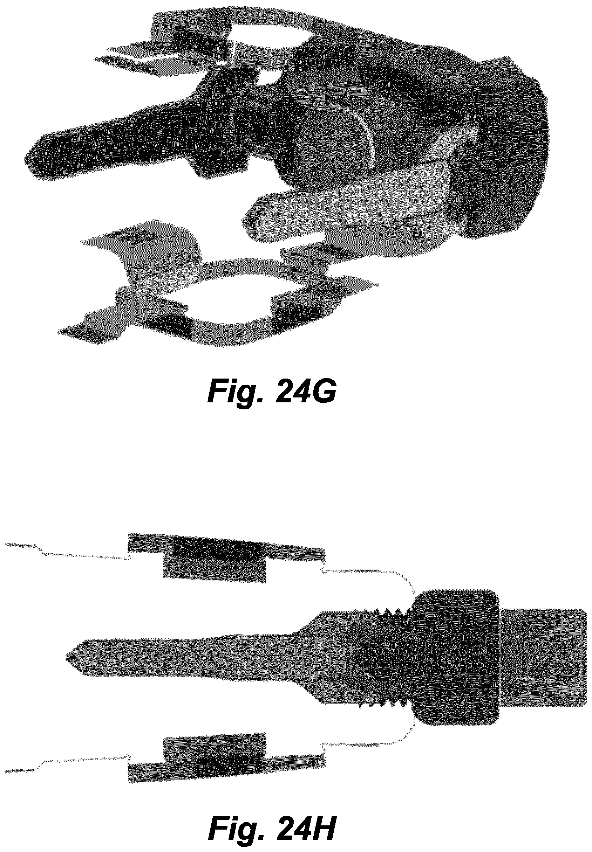

Figures 24A-I illustrate a smart interbody spacer, according to some embodiments of the present disclosure.

The smart interbody spacer, of Figures 24AI, is an expandable posterior lumbar interbody spacer with porous 3D printed titanium endplates and an integrated intelligent healing system that enables in situ bone growth stimulation and fusion monitoring. The interbody may be inserted at a contracted height as low as 6mm to reduce disruption during insertion and may provide up to 8mm of expansion and 22° of lordosis. The integrated intelligent healing system (also referred herein as the smart interbody spacer) may include several subsystems including electrochemical sensing, electrical stimulation, strain sensing, temperature sensing, and motion sensing. These subsystems may be separate subsystems or may be integrated into the same subsystem that is configured to perform different functions.

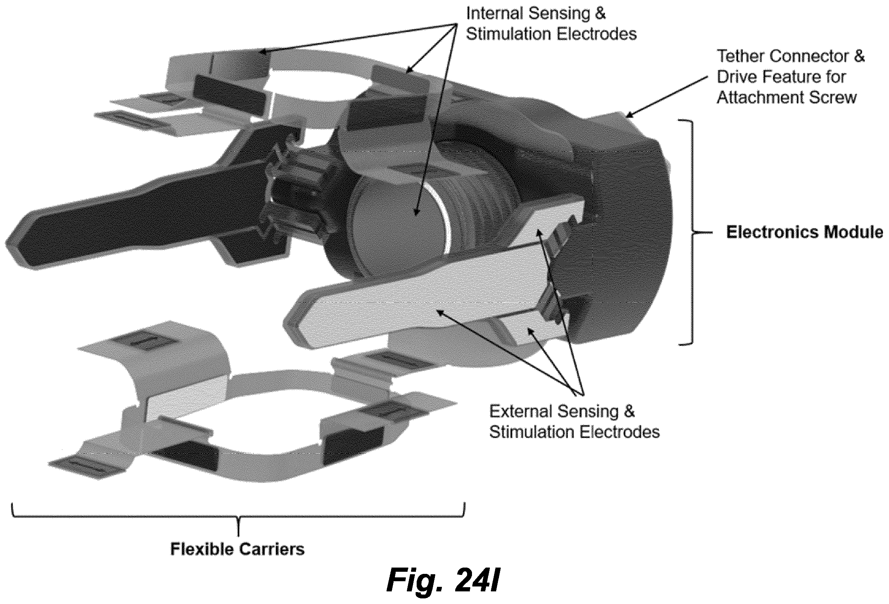

With reference to Figure 24I, the intelligent healing system includes an electronics module and carriers. The electronics module houses the circuit elements that enable electrochemical sensing and electrical stimulation functions. The carriers house the electrodes and distribute electrical signals between these elements and electronics module. The mechanical structure of the expandable interbody is hidden, in Figures 24G-I, to illustrate how the electronics module and carriers are integrated within the interbody structure.

A measurement process is described in further detail below.

As previously described in the microbial applications, each electrode in the multimodal electrode array may serve as the working electrode for electrochemical measurements. By cycling through the electrochemical measurement sequence at each electrode, a map of the electrochemical environment surrounding the electrode array can be constructed. This may enable spatial understanding of where cortical bone may be present versus not present in the interbody space. The map may also indicate a change of tissue adjacent to a set of electrodes in the array and a change in density of the tissue over time. For example, an EIS measurement mode may be performed, and the controller may be configured to generate a map indicating tissue characteristics across an area adjacent to the set of electrodes in the array and change of density of the tissue across the area adjacent to the set of electrodes in the array.

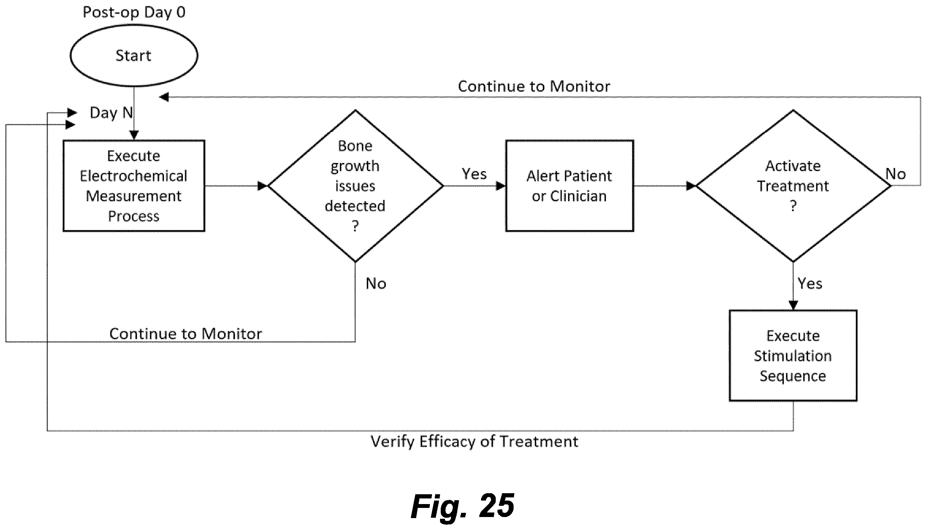

Figure 25 illustrates a flowchart of a stimulation sequence used in conjunction with a bone growth measurement sequence, according to some embodiments of the present disclosure.

In Figure 25, the measurement process may be designed to determine whether there is fusion (i.e. cortical bone) on or around the area of the implant. The measurement process could be executed automatically by the smart implant system in regular monitoring intervals such as once per day or once per week. Shorter intervals may be desired when the bone healing is most likely to occur. For example, the implant may wait to execute the measurement process until day 14 after postoperative inflammation has subsided and then continue measurement once per week from week 2 to week 52. After fusion is confirmed, the automatic measurements could be deactivated to conserve battery life or avoid patient compliance issues with wirelessly powering the device since the clinical value of fusion monitoring is no longer needed. In addition to automatic execution of the measurement process, measurements could also be manually initiated at any time by the patient or clinician using the connectivity system.

After the implant is implanted in the patient or a number of days (Day N) after, the implant device may execute electrochemical measurement process (including at least one measurement mode). Based on the measurements, the implant device (e.g., the controller in the implant device) may determine whether a bone growth issue is detected. For example, if a predetermined level of bone growth has not occurred by a certain time.

If a bone growth issue is not detected, then the implant device may start the process over again. If a bone growth issue is detected, then the implant device may alert the patient (e.g., by transmitting a message to a device of the patient) or the clinician (e.g., by transmitting a message to a clinician device directly, via a patient device, or via an external device that is used to transmit/receive message to/from the clinician). These alerts may include a message with an explicit indication of bone growth issue(s) and/or the measurements.

Then the implant device may determine to activate treatment (i.e., electrical stimulation). Alternatively, the clinician may determine to activate treatment and transmit (through a clinician device) instructions to the implant device to activate treatment.

If treatment is determined to be activated, then the implant device executes a stimulation sequence to promote bone growth responsive to the measurements obtained during the performed measurement mode(s) indicating an amount of bone growth, and the process is started over to verify efficacy of treatment. If treatment is determined to not be activated, then the process may start over.

A cleaning routine is discussed in further detail below.

The smart implant may be implanted in-situ for several years and the sensors will have several months of operation and occasional operation after that, therefore it may be necessary to occasionally replenish or clean the working (i.e. sensing) electrodes. Cleaning can be necessary because of biofouling or buildup of biological material on the surface of the electrode which causes reduction in sensitivity and increase in surface impedance. Embodiments discussed herein allow for the controller to control a 'cleaning routines' when the electrodes are considered fowled, corroded, and/or encapsulated they can be renewed by a series of potential and/or current steps or bursts. For example, based on the measurements obtained during measurement mode(s), the implant device may determine that electrodes are fowled, corroded, and/or encapsulated. Based on this, the controller may control the multiplexer to selectively connect different pairs of electrodes in an array (that includes the determined fowled/corroded/encapsulated electrode(s)) over time to perform a cleaning routine including electrically stimulating the different pairs of electrodes using a plurality of potential and/or current bursts.

A stimulation sequence is discussed in further detail below.

Electrical stimulation may be used as an effective treatment for stimulating osteogenic activity following musculoskeletal surgery. As stated earlier, any of the electrodes within the multimodal electrode array may be designated as anodes or cathodes for the purposes of electrical stimulation. Control over which electrodes serve as the anode and cathode (by the controller of the implant device) may enable steering of the electric field over the adjacent volume to direct the treatment energy. For example, treatment may be directed more towards areas with graft material as compared to areas of soft tissues. Sequential switching of the anode and cathode configuration may ensure that all adjacent volumes are sufficiently treated. This may be important for stimulation programs where the treatment is more effective at the cathode vs the anode or vise versa.

The stimulation sequence may be used independent of the fusion measurement process. For example, the patient or clinician may only want to leverage the bone growth treatment functions and not use the other smart implant functions. In these instances, the clinician has a non-invasive, highly directed treatment option that can be activated on-demand as a more effective alternative to wearable bone growth stimulator devices. Depending on the treatment protocol and program, the patient may experience accelerated bone growth with only one treatment session per day.

The stimulation sequence (i.e. treatment session) can also be used in conjunction with the measurement process to enable a sensing and treatment control loop. For example, the measurements from the first 6 weeks post-surgery may indicate delayed bone healing. The clinician could choose to more closely monitor the patient before administering treatment, or a treatment could be activated with the smart implant as a treatment measure. This clinical monitoring and treatment loop is described and illustrated in Figure 25.

Combined applications are discussed in further detail below.

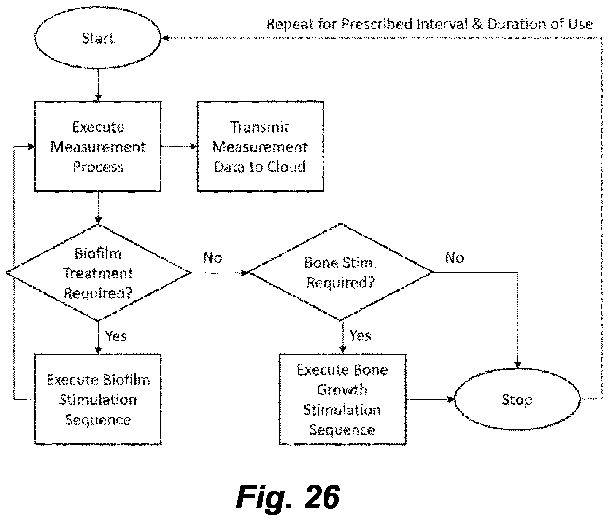

The previously described systems for microbial and osteogenic applications may be combined into a singular system. A single multimodal electrode array may be used for the purposes of electrochemical sensing and electrical stimulation of biofilm and tissue. As mentioned, each electrode in the multimodal array can act as the working electrode, counter electrode, reference electrode, anode, or cathode depending on the programming of the system. Switching between functions across the entire electrode array enables steerable, full-coverage stimulation as well as spatial electrochemical measurements. One example of how these measurement and treatment processes could be combined into a single automated process is illustrated in Figure 26.

Figure 26 illustrates a flowchart of a combined system of osteogenic and microbial applications, according to some embodiments of the present disclosure.

The process may start, and the implant device may execute a measurement process. For example, a controller of the implant device may control a multiplexer of the implant device to selectively connect different pairs of electrodes in an array over time to perform measurement modes that include at least two of: open circuit potential (OCP) measurement, where measurement circuitry measures free potential through a first pair of electrodes when the controller does not apply current between the first pair of electrodes; amperometry measurement, where the controller applies power to one of the electrodes in a second pair of electrodes and the measurement circuitry measures the resulting current at the other electrode in the second pair of electrodes; and electrochemical impedance spectroscopy (EIS) measurement, where the controller applies an alternating current to one of the electrodes in a third pair of electrodes and the measurement circuitry measures the resulting current at the other electrode in the third pair of electrodes.

The measurement data obtained by the performance of the measurement modes may be transmitted to a cloud that is accessible to the patient that the implant device is implanted into and/or a clinician.

The implant device may determine if biofilm has formed on or near one of the electrodes based on the measurements. If the implant device determines that biofilm has formed, then the implant device may determine that biofilm treatment (i.e., electrical stimulation) is needed. If so, then the implant device executes a biofilm stimulation sequence. If not, then the implant device determines if bone stimulation is needed based on the measurements (e.g., whether a level of bone density is measured during measurements of impedance by a pair of electrodes). If the implant device determines that bone stimulation is not required, then the process may stop. Optionally, instead of stopping the process, the implant device may start the process over from the beginning for, either immediately or after a predefined time period.

If the implant device determines that bone stimulation is required, then the implant device may execute a bone growth stimulation sequence, which may be different than the biofilm stimulation sequence.

As described herein (as part of other embodiments and processes or as a stand alone process/embodiment), a method may be performed by a controller of the implant device implantable within a body of a patient. The method may include controlling a multiplexer to selectively connect different pairs of electrodes, of a set of electrodes spaced apart on the implant device, to measurement circuity over time to perform measurement modes that include at least two of: open circuit potential (OPC) measurement, where measurement circuitry of the implant device measures free potential through a first pair of electrodes while the controller does not apply current between the first pair of electrodes; amperometry measurement, where the controller applies power to one of the electrodes in a second pair of electrodes while the measurement circuitry measures the current resulting at the other electrode in the second pair of electrodes; and electrochemical impedance spectroscopy (EIS) measurement, where the controller applies an alternating current to one of the electrodes in a third pair of electrodes while the measurement circuitry measures the current resulting at the other electrode in the third pair of electrodes. This method may further include the controller controlling electrical stimulation of at least one pair of electrodes based on the measurements. For example, the electrical stimulation may be in response to the measurements indicating bone growth issue, infection (e.g., biofilm development), etc.

### Further Definitions and Embodiments:

In the above-description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the spirit and scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

The invention could be defined inter alia by the following examples
1. An implant device implantable within a body of a patient, comprising: a set of electrodes in an array spaced apart on the implant device; measurement circuitry configured to perform measurement modes on signals from the electrodes; a multiplexer that interconnects different pairs of electrodes in the array to the measurement circuitry; and a controller that controls the multiplexer to selectively connect different pairs of electrodes in the array over time to perform the measurement modes that include at least two of: open circuit potential (OCP) measurement, wherein the measurement circuitry measures free potential through a first pair of electrodes while the controller does not apply current between the first pair of electrodes; amperometry measurement, wherein the controller applies power to one of the electrodes in a second pair of electrodes while the measurement circuitry measures current resulting at the other electrode in the second pair of electrodes; and electrochemical impedance spectroscopy (EIS) measurement, wherein the controller applies an alternating current to one of the electrodes in a third pair of electrodes while the measurement circuitry measures current resulting at the other electrode in the third pair of electrodes.
2. The implant device of Claim 1, wherein the at least two measurement modes are performed sequentially during separate time windows, and the first, second, and third pair of electrodes are the same pair of electrodes.
3. The implant device of Example 2, wherein between a sequence of two of the measurement modes, the controller is configured to delay a time period during which the controller applies no current or a reverse biased current, that is a reverse bias of the current that was applied during a prior completed measurement mode, to the pair of electrodes used during the prior completed measurement mode.
4. The implant device of Example 1, wherein a first measurement mode of the at least two measurement modes is performed during an at least partially overlapping time window while a second measurement mode of the at least two measurement modes is performed, and the pair of electrodes that is used in the first measurement mode is different than the pair of electrodes that is used in the second measurement mode.
5. The implant device of Example 1, wherein the controller further controls supply of a direct voltage to one pair of the electrodes in the array and controls supply of an alternating current swept across a range of frequencies to another pair of the electrodes in the array.
6. The implant device of Example 1, further comprising: another set of electrodes in another array spaced apart on the implant device and physically separate and electrically isolated from the set of electrodes in the array, wherein the controller controls the multiplexer to selectively connect different pairs of electrodes in the another array over time to sequentially perform the measurement modes.
7. The implant device of Example 1, wherein during a first time window the controller controls the multiplexer to select a first electrode in the pair of electrodes to operate as a working electrode and a second electrode in the pair of electrodes to operate as a counter electrode.
8. The implant device of Example 7, wherein during a non-overlapping time window relative to the first time window the controller controls the multiplexer to select the first electrode to operate as the counter electrode and selects a third electrode that is different than the second electrode to operate as the working electrode.
9. The implant device of Example 1, wherein the controller further generates a measurement report indicating results of the performed measurement modes, and controls transmission of the report to a device that is external to the body of the patient.
10. The implant device of Example 1, wherein the controller controls the multiplexer to sequentially select each electrode in the set of electrodes in the array, during different time windows, to operate as a working electrode while the controller sequentially performs the at least two of the measurement modes.
11. The implant device of Example 1, wherein the at least two measurement modes are repetitively performed by the controller at a time interval electronically settable in memory of the implant device.
12. The implant device of Example 1, wherein the at least two measurement modes are performed by the controller responsive to expiration of a time period measured since implantation of the implant device within the body of the patient.
13. The implant device of Example 1, wherein the controller further controls the multiplexer to selectively connect different pairs of electrodes in the array over time to perform a cleaning routine including electrically stimulating the different pairs of electrodes using a plurality of electrical potential and/or current bursts.
14. The implant device of Example 1, wherein one of the at least two measurement modes performed includes EIS measurement and the controller is configured to generate a map indicating tissue characteristics across an area adjacent to the set of electrodes in the array and change of density of the tissue across the area adjacent to the set of electrodes in the array.
15. The implant device of Example 1, wherein one of the at least two measurement modes performed includes EIS measurement and the controller is configured to perform electrical impedance tomography (EIT) using the measurements taken during the EIS measurement to construct a three-dimensional (3D) model of tissue location and characteristics adjacent the set of electrodes.
16. The implant device of Example 1, wherein one of the at least two measurement modes performed includes EIS measurement and wherein the controller further controls a cathodic biased electrical stimulation of the different pairs of electrodes in the array responsive to measurements taken during the EIS measurement indicative of amount of bone growth.
17. The implant device of Example 1, wherein the implant device is an interbody spacer and the controller is housed within the interbody spacer, and wherein the controller further controls electrical stimulation of the different pairs of electrodes on a surface of the interbody spacer to promote bone growth responsive to the measurements obtained during at least one of the performed measurement modes indicating an amount of bone growth.
18. A method performed by a controller of an implant device implantable within a body of a patient, comprising: controlling a multiplexer to selectively connect different pairs of electrodes, of a set of electrodes spaced apart on the implant device, to measurement circuitry over time to perform measurement modes that include at least two of: open circuit potential (OPC) measurement, wherein measurement circuitry of the implant device measures free potential through a first pair of electrodes while the controller does not apply current between the first pair of electrodes; amperometry measurement, wherein the controller applies power to one of the electrodes in a second pair of electrodes while the measurement circuitry measures current resulting at the other electrode in the second pair of electrodes; and electrochemical impedance spectroscopy (EIS) measurement, wherein the controller applies an alternating current to one of the electrodes in a third pair of electrodes while the measurement circuitry measures current resulting at the other electrode in the third pair of electrodes.
19. The method of Example 18, wherein the at least two measurement modes are performed sequentially during separate time windows and the first, second, and third pair of electrodes are the same pair of electrodes.
20. The implant device of Example 18, wherein a first measurement mode of the at least two measurement modes is performed during an at least partially overlapping time window while a second measurement mode of the at least two measurement modes is performed, and the pair of electrodes that is used in the first measurement mode is different than the pair of electrodes that is used in the second measurement mode.

## Claims

1. An implant device implantable within a body of a patient, comprising:
a set of electrodes in an array spaced apart on the implant device;
measurement circuitry configured to perform measurement modes on signals from the electrodes;
a multiplexer that interconnects different pairs of electrodes in the array to the measurement circuitry; and
a controller that controls the multiplexer to selectively connect different pairs of electrodes in the array over time to perform the measurement modes that include at least two of:
open circuit potential (OCP) measurement, wherein the measurement circuitry measures free potential through a first pair of electrodes while the controller does not apply current between the first pair of electrodes;
amperometry measurement, wherein the controller applies power to one of the electrodes in a second pair of electrodes while the measurement circuitry measures current resulting at the other electrode in the second pair of electrodes; and
electrochemical impedance spectroscopy (EIS) measurement, wherein the controller applies an alternating current to one of the electrodes in a third pair of electrodes while the measurement circuitry measures current resulting at the other electrode in the third pair of electrodes.

2. The implant device of Claim 1, wherein the at least two measurement modes are performed sequentially during separate time windows, and the first, second, and third pair of electrodes are the same pair of electrodes.

3. The implant device of Claim 2, wherein between a sequence of two of the measurement modes, the controller is configured to delay a time period during which the controller applies no current or a reverse biased current, that is a reverse bias of the current that was applied during a prior completed measurement mode, to the pair of electrodes used during the prior completed measurement mode.

4. The implant device of any one of the preceding claims, wherein a first measurement mode of the at least two measurement modes is performed during an at least partially overlapping time window while a second measurement mode of the at least two measurement modes is performed, and the pair of electrodes that is used in the first measurement mode is different than the pair of electrodes that is used in the second measurement mode.

5. The implant device of any one of the preceding claims, wherein the controller further controls supply of a direct voltage to one pair of the electrodes in the array and controls supply of an alternating current swept across a range of frequencies to another pair of the electrodes in the array.

6. The implant device of any one of the preceding claims, further comprising:
another set of electrodes in another array spaced apart on the implant device and physically separate and electrically isolated from the set of electrodes in the array,
wherein the controller controls the multiplexer to selectively connect different pairs of electrodes in the another array over time to sequentially perform the measurement modes.

7. The implant device of any one of the preceding claims, wherein during a first time window the controller controls the multiplexer to select a first electrode in the pair of electrodes to operate as a working electrode and a second electrode in the pair of electrodes to operate as a counter electrode.

8. The implant device of Claim 7, wherein during a non-overlapping time window relative to the first time window the controller controls the multiplexer to select the first electrode to operate as the counter electrode and selects a third electrode that is different than the second electrode to operate as the working electrode.

9. The implant device of Claim 1, wherein the controller further generates a measurement report indicating results of the performed measurement modes, and controls transmission of the report to a device that is external to the body of the patient.

10. The implant device of any one of the preceding claims, wherein the controller controls the multiplexer to sequentially select each electrode in the set of electrodes in the array, during different time windows, to operate as a working electrode while the controller sequentially performs the at least two of the measurement modes.

11. The implant device of any one of the preceding claims, wherein the at least two measurement modes are repetitively performed by the controller at a time interval electronically settable in memory of the implant device.

12. The implant device of any one of the preceding claims, wherein the at least two measurement modes are performed by the controller responsive to expiration of a time period measured since implantation of the implant device within the body of the patient.

13. The implant device of any one of the preceding claims, wherein the controller further controls the multiplexer to selectively connect different pairs of electrodes in the array over time to perform a cleaning routine including electrically stimulating the different pairs of electrodes using a plurality of electrical potential and/or current bursts.

14. The implant device of any one of the preceding claims, wherein one of the at least two measurement modes performed includes EIS measurement and the controller is configured to generate a map indicating tissue characteristics across an area adjacent to the set of electrodes in the array and change of density of the tissue across the area adjacent to the set of electrodes in the array and wherein preferably one of the at least two measurement modes performed includes EIS measurement and the controller is configured to perform electrical impedance tomography (EIT) using the measurements taken during the EIS measurement to construct a three-dimensional (3D) model of tissue location and characteristics adjacent the set of electrodes and wherein preferably one of the at least two measurement modes performed includes EIS measurement and wherein the controller further controls a cathodic biased electrical stimulation of the different pairs of electrodes in the array responsive to measurements taken during the EIS measurement indicative of amount of bone growth15. The implant device of any one of the preceding claims, wherein the implant device is an interbody spacer and the controller is housed within the interbody spacer, and wherein the controller further controls electrical stimulation of the different pairs of electrodes on a surface of the interbody spacer to promote bone growth responsive to the measurements obtained during at least one of the performed measurement modes indicating an amount of bone growth.
